# EUROPEAN PATENT SPECIFICATION

(11) **EP 1 614 686 B1**
(45) Date of publication and mention of the grant of the patent: **07.05.2014**
(21) Application number: 04727422.0
(22) Date of filing: 14.04.2004
(51) Int. Cl.: C07D 403/06

(54) **BROMIDE AND ITS CRYSTAL**
BROMID UND DESSEN KRISTALL
BROMURE ET CRISTAL DE BROMURE

(30) Priority: 15.04.2003 JP 2003109793
(43) Date of publication of application: 11.01.2006
(73) Proprietor: Astellas Pharma Inc., Tokyo 103-8411 (JP)
(72) Inventor: KINOYAMA, Isao, 2-chome, Chuo-ku Tokyo 1038411 (JP); SAKAMOTO, Kenichirou, 2-chome, Chuo-ku Tokyo 1038411 (JP); OKUI, Hiroki, 2-chome, Chuo-ku Tokyo 1038411 (JP); HAMADA, Noritaka, 2-chome, Chuo-ku Tokyo 1038411 (JP); MATSUHISA, Akira, 2-chome, Chuo-ku Tokyo 1038411 (JP)
(74) Representative: Bates, Philip Ian
(86) International application number: PCT/JP2004/005321
(87) International publication number: WO 2004/092160

(56) References cited:
- EP-A1- 1 256 576
- WO-A1-01/60803
- JP-A- 2003 128 548

## Description

### Technical Field

The present invention relates to 1-(2-methoxyethyl)-2-methyl-4,9-dioxo-3-(pyrazin-2-ylmethyl)-4,9-dihydro-1H-naphtho[2,3-d]imidazol-3-ium bromide which is a novel compound useful as medicines, particularly to its crystals.

### Background of the Invention

Fused imidazolium derivatives which are expected as candidates of antitumor agents having good antitumor activity, low toxicity and wide safety margins are disclosed in the pamphlet of International Publication 01/60803. Particularly, the 1-(2-methoxyethyl)-2-methyl-4,9-dioxo-3-(pyrazin-2-ylmethyl)-4,9-dihydro-1H-naphtho[2,3-d]imidazol-3-ium chloride disclosed in Example 154 is a compound which is expected as an antitumor agent, because it has good in vivo tumor growth inhibitory activity and low toxicity (cf. page 22 of said pamphlet).

The 1-(2-methoxyethyl)-2-methyl-4,9-dioxo-3-(pyrazin-2-ylmethyl)-4,9-dihydro-1H-naphtho[2,3-d]imidazol-3-ium chloride (to be referred to as chloride hereinafter) described in the aforementioned pamphlet is a compound on which the 1-position and 3-position of the imidazole ring are respectively substituted by substituted lower alkyl, thereby forming imidazolium cation, and said cation forms an ion pair with chlorine anion. It is described that said compound has tautomers shown by the following formula due to delocalization of the cation.

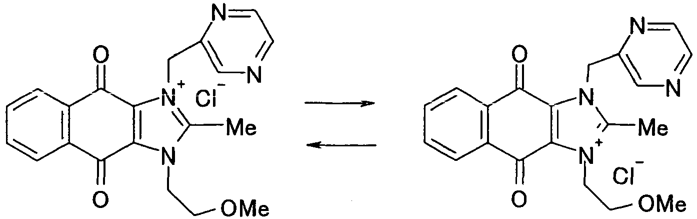

The aforementioned chloride was obtained as a crystalline anhydride, but it is unstable against humidity due to its hygroscopic property, such as easy formation of crystal transition between monohydrate and anhydride depending on the humidity condition and deliquescence under a high humidity condition, as well as increase of degradation products at the time of long-term storage and the like, so that it was extremely difficult to industrially produce a medicine using this as the production material.

The inventors of the invention have conducted intensive studies with the aim of providing 1-(2-methoxyethyl)-2-methyl-4,9-dioxo-3-(pyrazin-2-ylmethyl)-4,9-dihydro-1H-naphtho[2,3-d]imidazol-3-ium having suitable properties as the bulk for medicines.

### Disclosure of the Invention

The present inventors have accomplished the invention by unexpectedly finding that novel 1-(2-methoxyethyl)-2-methyl-4,9-dioxo-3-(pyrazin-2-ylmethyl)-4,9-dihydro-1H-naphtho[2,3-d]imidazol-3-ium bromide (to be referred to as bromide hereinafter) having bromine anion as the counter anion has suitable properties as a bulk for medicines. Particularly, it was quite unexpected that the bromide of the invention would have polymorphs, and both of them would not have hygroscopic property but have good storage stability.

That is, the invention relates to 1-(2-methoxyethyl)-2-methyl-4,9-dioxo-3-(pyrazin-2-ylmethyl)-4,9-dihydro-1H-naphtho[2,3-d]imidazol-3-ium having suitable properties as a bulk for medicines, particularly to its crystals. The following two crystal forms are present in the bromide of the invention, and these crystals are included in the invention.

α-form Crystal: A crystal having powder X-ray diffraction peaks of 2θ (°) = 8.5, 14.8, 19.7, 25.7, 30.2. Preferably, a crystal having a heat absorption peak of about 210°C, namely from 207 to 213°C, by DSC analysis.

β-form Crystal: A crystal having powder X-ray diffraction peaks of 2θ (°) = 9.2, 12.6, 14.6, 18.0, 21.1, 24.9, 26.4, 27.1. Preferably, a crystal having a heat absorption peak of about 204°C, namely from 201 to 207°C, by DSC analysis.

### Brief Description of the Drawings

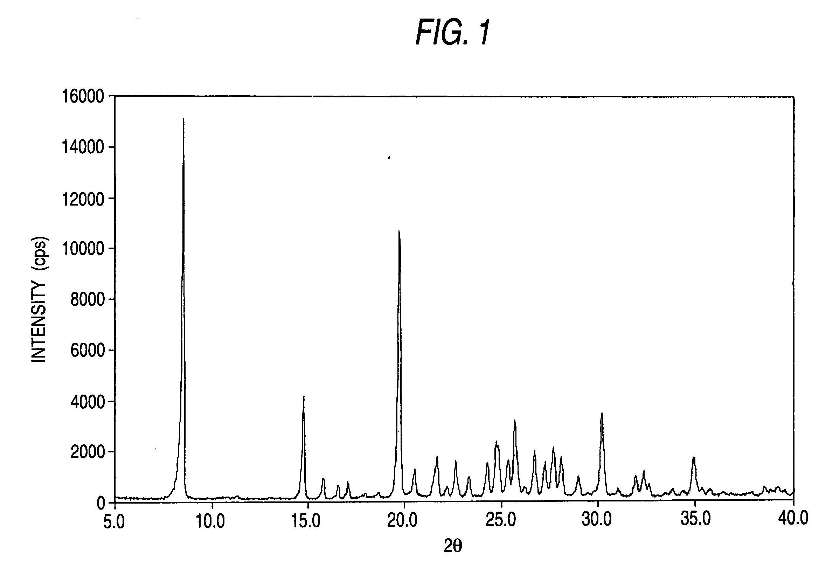

Fig. 1 is a graph showing powder X-ray diffraction of α-form Crystal of 1-(2-methoxyethyl)-2-methyl-4,9-dioxo-3-(pyrazin-2-ylmethyl)-4,9-dihydro-1H-naphtho[2,3-d]imidazol-3-ium bromide.

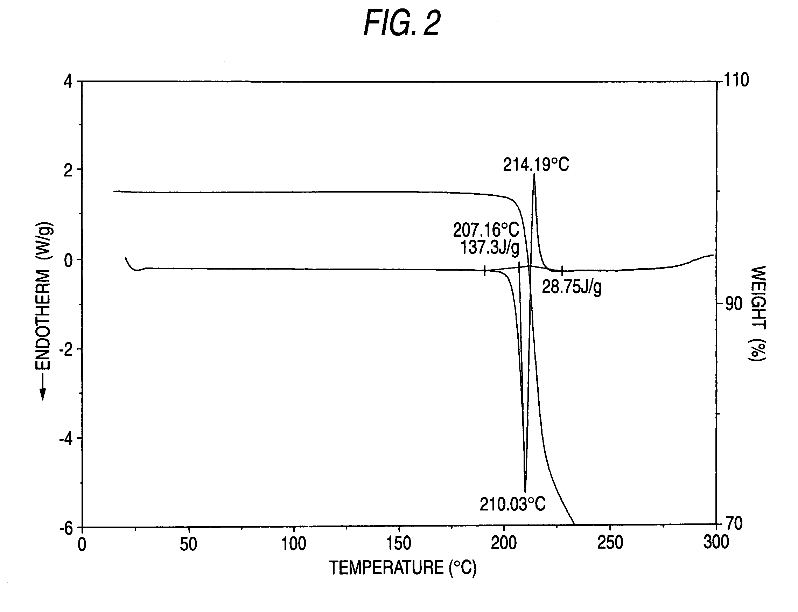

Fig. 2 is a graph showing thermal analysis of α-form Crystal of 1-(2-methoxyethyl)-2-methyl-4,9-dioxo-3-(pyrazin-2-ylmethyl)-4,9-dihydro-1H-naphtho[2,3-d]imidazol-3-ium bromide.

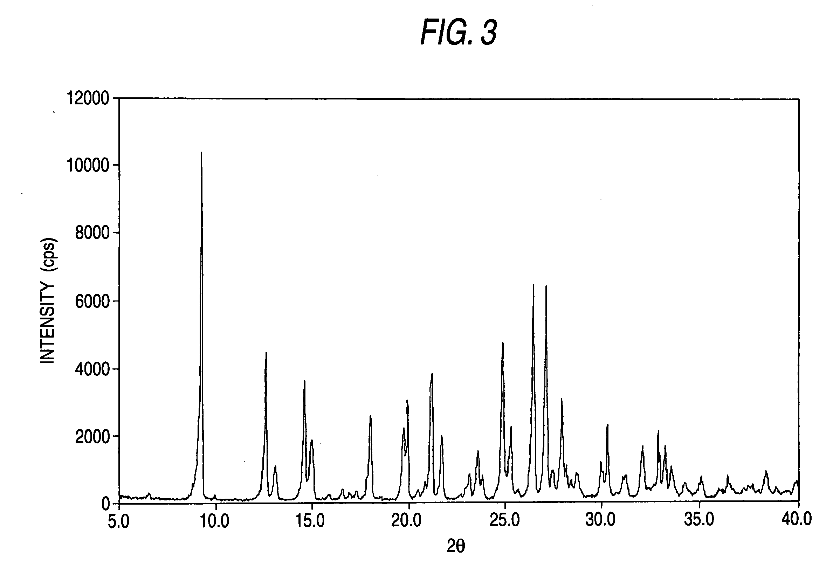

Fig. 3 is a graph showing powder X-ray diffraction of β-form Crystal of 1-(2-methoxyethyl)-2-methyl-4,9-dioxo-3-(pyrazin-2-ylmethyl)-4,9-dihydro-1H-naphtho[2,3-d]imidazol-3-ium bromide.

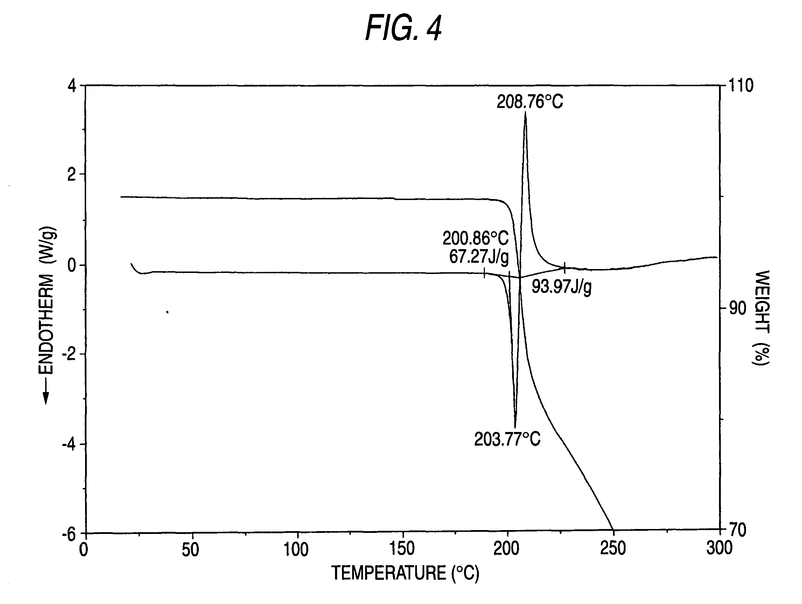

Fig. 4 is a graph showing thermal analysis of β-form Crystal of 1-(2-methoxyethyl)-2-methyl-4,9-dioxo-3-(pyrazin-2-ylmethyl)-4,9-dihydro-1H-naphtho[2,3-d]imidazol-3-ium bromide.
Fig. 5 is a graph showing water absorption/desorption isothermal curve of β-form Crystal of 1-(2-methoxyethyl)-2-methyl-4,9-dioxo-3-(pyrazin-2-ylmethyl)-4,9-dihydro-1H-naphtho[2,3-d]imidazol-3-ium bromide.
Fig. 6 is a graph showing water absorption/desorption isothermal curve of 1-(2-methoxyethyl)-2-methyl-4,9-dioxo-3-(pyrazin-2-ylmethyl)-4,9-dihydro-1H-naphtho[2,3-d]imidazol-3-ium chloride.

### Best Mode for Carrying Out the Invention

The following describes the invention in detail.

Regarding the bromide of the invention, it may be any substance which is stable in such a degree that it can be used as a bulk for medicines, and the α- or β-form Crystal which has no hygroscopic property and is stable as described in the following is desirable. Particularly preferred is β-form Crystal.

The bromide of the invention has no hygroscopic property and is chemically stable during its storage for a prolonged period of time. While transition of the α-form Crystal to the β-form Crystal can be found only under an extremely high humidity condition, the β-form Crystal is a crystal which does not cause crystal transition and is also physically stable. It was confirmed that each of the α-and β-form Crystals is stable for a period of 3 months at 40°C under a relative humidity of 75% and is suitable as a bulk for medicines, particularly as a bulk for solid pharmaceutical preparations.

In this connection, each of the crystals is characterized by the respective powder X-ray diffraction spectrum [2θ (°)], but this should not be understood strictly, because from the viewpoint of properties of powder X-ray diffraction data, crystal lattice spacing and general pattern are important in finding identity of the crystal, and the relative strength can vary to some extent depending on the direction of crystal growth, size of particles and measuring conditions.

According to the studies carried out by the present inventors, search for polymorphs was intensively conducted also on the chloride, but it was not able to find a crystal having properties useful as a material of medicines. Contrary to this, each of the thus found polymorphs of the novel bromide of the invention unexpectedly showed suitable properties useful as a material of medicines, which rendered the development of 1-(2-methoxyethyl)-2-methyl-4,9-dioxo-3-(pyrazin-2-ylmethyl)-4,9-dihydro-1H-naphtho[2,3-d]imidazol-3-ium for the first time as a medicine.

### (Production methods)

The bromide of the invention can be produced by the reaction shown by the following formula.

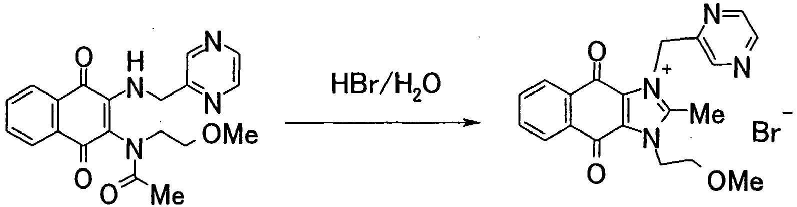

The reaction can be carried out, for example, by employing the method described in J. Org. Chem. USSR, 1, 1479 - 85 (1965), and it is advantageous to carry out the reaction at ambient temperature or under heating in an appropriate inert solvent (e.g., an alcohol solvent), using a reaction corresponding amount or an excess amount of hydrobromic acid.

The crystals of the bromide of the invention can be separately produced with good reproducibility by selecting the crystallization solvent as described in the following, which is advantageous in producing them by industrial production.

The α-form Crystal can be obtained with good reproducibility when recrystallized in ethanol, ethanol/water, 1-propanol/water, 2-propanol/water or acetone/water. In addition, when the β-form Crystal is stirred in the aforementioned solvent, it can be transitioned to the α-form Crystal.

On the other hand, the β-form Crystal can be obtained with good reproducibility when recrystallized in methanol, water, acetonitrile/water or methanol/acetonitrile. In addition, when the α-form Crystal is stirred in the aforementioned solvent, it can be transitioned to the β-form Crystal.

The bromide of the invention can be used for the production of a medicine as the bulk for the medicine, in combination with carriers, fillers and the like for pharmaceutical preparation use generally used in this field. Production of medicines can be carried out by the methods generally used in this field.

The pharmaceutical preparations comprising the compound of the invention may be in the form of either oral administration preparations by tablets, pills, capsules, granules, powders, solutions or the like, or parenteral administration preparations by intraarticular, intravenous, intramuscular or the like injections, suppositories, solutions for percutaneous use, ointments, adhesive preparations for percutaneous use, transmucosal solutions, transmucosal adhesive preparations, inhalations or the like. Particularly, tablets, pills, capsules, granules, powders and the like for oral administration and inhalations, transnasal preparations and the like for parenteral administration, which use crystals of the bromide as the production material, are advantageous as stable solid preparations.

In the solid preparations for oral administration, the compound of the invention is mixed with at least one inert filler such as lactose, mannitol, glucose, hydroxypropylcellulose, microcrystalline cellulose, starch, polyvinyl pyrrolidone, aluminum magnesium silicate or the like. In accordance with the usual way, the composition may contain inert additives such as magnesium stearate or the like lubricant, carboxymethylstarch sodium or the like disintegrating agent and solubilizing agent. As occasion demands, tablets or pills may be coated with a sugar coating or a gastric or enteric coating agent.

The liquid composition for oral administration includes pharmaceutically acceptable emulsions, solutions, suspensions, syrups, elixirs and the like, and contains a generally used inert solvent such as purified water or ethanol. In addition to the inert solvent, this composition may also contain a solubilizing agent, a moistening agent, a suspending agent and the like auxiliary agents, as well as sweeteners, correctives, aromatics and antiseptics.

The injections for parenteral administration includes aseptic aqueous or non-aqueous solutions, suspensions and emulsions. Examples of the aqueous solvent include distilled water for injection and physiological saline. Examples of the non-aqueous solvent include propylene glycol, polyethylene glycol, olive oil or the like plant oil, ethanol or the like alcohol, polysorbate 80 (trade name) and the like. Such a composition may further contain a tonicity agent, an antiseptic, a moistening agent, an emulsifying agent, a dispersing agent, a stabilizing agent and a solubilizing agent. These are sterilized by filtration through a bacteria retaining filter, blending of a germicide or irradiation. Alternatively, they may be used by producing a sterile solid composition and dissolving it in sterile water or a sterile solvent for injection prior to its use.

The following illustratively describes the invention based on examples.

MAC Science MXP18TAHF22 was used in the measurement of powder X-ray diffraction, and the measurement was carried out under conditions of X-ray tube: Cu, tube current: 40 mA, tube voltage: 40 kV, sampling width: 0.020°, scanning rate: 3°/min, wavelength: 1.54056 A, and measuring range of diffraction angle (2θ): 5 to 40°.

Regarding the thermal analyses (DSC and TGA), the measurements were carried out under the following respective conditions.

DSC: DSC 2910 Differential Scanning Calorimeter manufactured by TA Instruments, from room temperature to 300°C (10°C/min), N₂ (50 ml/min), aluminum sample pan.

TGA: TGA 2950 Thermogravimetric Analyzer manufactured by TA Instruments, from room temperature to 300°C (10°C/min), N₂ (50 ml/min), platinum sample pan.

### Example 1

Ethanol (10 ml) suspension of N-{1,4-dioxo-3-[(pyrazin-2-ylmethyl)amino]-1,4-dihydronaphthalen-2-yl}-N-(2-methoxyethyl)acetamide (2.0 g) was mixed with 47% hydrobromic acid (1 ml) and stirred at 50°C for 38 hours. After completion of the reaction, the reaction mixture was cooled to room temperature to find precipitation of crystals. This was stirred for 5 hours, and then the crystals were collected by filtration, washed with ethanol and dried to obtain the bromide (α-form Crystal) (2.01 g) as orange crystals.

### Example 2

Ethanol (680 ml) suspension of N-{1,4-dioxo-3-[(pyrazin-2-ylmethyl)amino]-1,4-dihydronaphthalen-2-yl}-N-(2-methoxyethyl)acetamide (170 g) was mixed with 47% hydrobromic acid (85 ml) and stirred by heating at 60°C for 13 hours. At the time of completing the reaction, a small amount of insoluble matter was formed. When this was heat-dissolved by adding ethanol (170 ml) and then spontaneously cooled, crystals were precipitated. They were stirred at room temperature for 24 hours and then collected by filtration and washed with ethanol to obtain 185 g (wet weight) of crude crystals. The crude crystals were recrystallized using a mixed solvent of ethanol (850 ml) and water (85 ml) to obtain the bromide (α-form Crystal) (104.80 g).

The residue (53.6 g) obtained by concentrating the recrystallization mother liquor was mixed with water (250 ml) and dissolved therein at room temperature. The thus formed insoluble matter was removed by filtration, and the filtrate was concentrated under a reduced pressure to obtain a yellow crystalline residue (53.5 g). The residue was mixed with ethanol (250 ml), suspended and stirred at 50°C for 1 hour and then stirred at room temperature for 21 hours. The crystals were collected by filtration and then washed with ethanol and dried to obtain the bromide (β-form Crystal) (47.34 g).

### Example 3

Methanol (25 ml) suspension of N-{1,4-dioxo-3-[(pyrazin-2-ylmethyl)amino]-1,4-dihydronaphthalen-2-yl}-N-(2-methoxyethyl)acetamide (5 g) was mixed with 47% hydrobromic acid (2.5 ml) and heated under reflux for 5 hours. After completion of the reaction, methanol (15 ml) was evaporated under ordinary pressure. When the residue was spontaneously cooled, crystals were precipitated. They were stirred at room temperature for 15 hours and then mixed with ethyl acetate (40 ml) and further stirred at room temperature for 7 hours. The crystals were collected by filtration, washed and dried to obtain the bromide (P-form Crystal) (4.61 g) as yellow crystals.

### Example 4

(1) Methanol (15 ml) was added to the α-form Crystals (5 g) and stirred at room temperature. With the progress of stirring, color of the crystals changed from orange to yellow, showing their transition from α-form to β-form. After 27 hours, the crystals were collected by filtration and dried at 50°C to obtain β-form Crystals (2.88 g).
(2) By using α-form Crystals (2 g) and water (2 ml) and carrying out the same operation of (1), β-form Crystals (1.48 g) were obtained.
(3) By using α-form Crystals (0.5 g), acetonitrile (2.5 ml) and water (0.125 ml) and carrying out the same operation of (1), β-form Crystals (0.28 g) were obtained.
(4) By using α-form Crystals (2 g), acetonitrile (10 ml) and methanol (2 ml) and carrying out the same operation of (1), β-form Crystals (0.77 g) were obtained.
(5) By dissolving α-form Crystals (5 g) in methanol (10 ml) with heating and stirring this at room temperature for 0.5 hour, a yellow slurry of β-form Crystals were obtained. Ethyl acetate (40 ml) was further added thereto, and after 16 hours of stirring, the crystals were collected by filtration and dried to obtain β-form Crystals (4.54 g).

### Example 5

(1) β-form Crystals (5 g) were dissolved in ethanol (45 ml) with heating, spontaneously cooled and then stirred at room temperature for 5 hours. The crystals were collected by filtration and dried at 50 to 60°C under a reduced pressure to obtain α-form Crystals (4.47 g).
(2) By using α-form Crystals (2 g), acetone (10 ml) and water (2.5 ml) and carrying out the same operation of (1), α-form Crystals (0.95 g) were obtained.
(3) By using α-form Crystals (1.1 g), 2-propanol (5 ml) and water (1 ml) and carrying out the same operation of (1), α-form Crystals (0.85 g) were obtained.
(4) By using α-form Crystals (1 g), 1-propanol (5 ml) and water (0.5 ml) and carrying out the same operation of (1), α-form Crystals (0.71 g) were obtained.

### Reference Example (Production example of the chloride as a comparative compound)

Ethanol (30 ml) suspension of N-{1,4-dioxo-3-[(pyrazin-2-ylmethyl)amino]-1,4-dihydronaphthalen-2-yl}-N-(2-methoxyethyl)acetamide (2.51 g) was mixed with concentrated hydrochloric acid (7.5 ml) and stirred at room temperature for 18 hours. To the reaction mixture, ethanol (10 ml) and concentrated hydrochloric acid (2.5 ml) were further added and the mixture was stirred at room temperature for 5 hours and then at 50 to 55°C for 3 hours. After concentration of the reaction mixture under a reduced pressure, the residue was dissolved in a large amount of ethanol, and the insoluble matter was removed by filtration. The mother liquor was concentrated under a reduced pressure, the residue was dissolved in ethanol (15 ml) with heating, and then mixed with ethyl acetate (30 ml) and stirred at room temperature, and the thus precipitated crystals were collected by filtration. The mother liquor was concentrated under a reduced pressure, and then the residue (1.67 g) was purified by an ODS column chromatography (YMC GEL ODS-A 120-S150). A water-methanol (50:1) eluate was concentrated under a reduced pressure, the residue was dissolved in ethanol (5 ml) with heating, mixed with ethyl acetate (25 ml) and stirred at room temperature, and the thus precipitated crystals were collected by filtration, washed and dried to obtain the chloride (696 mg) as yellow crystals. This was possessed of the same physicochemical properties of the chloride described in Example 154 of WO01/60803.

Effects of the bromide of the invention are shown in the following.

### Test Example 1 (Evaluation of hygroscopic property)

### (Test method)

About 0.5 g of each of the α-form Crystals and β-form Crystals of the bromide obtained in Example 2 was put into a weighing bottle of known mass and, after precisely calculating its mass, preserved under the following storage condition at 25°C to measure changes in the mass.

Storage condition: A desiccator adjusted to a humidity of about 0% R.H. (silica gel), 33% R.H. (saturated MgCl₂·6H₂O aqueous solution), 51% R.H. (saturated Ca(NO₃)₂ aqueous solution), 75% R.H. (saturated Nacl aqueous solution), or 93% R.H. (saturated KNO₃ aqueous solution).
i) Moisture absorption (dehydrated amount): By taking out each sample after 7 days of the storage, the weight change was precisely measured and recorded to calculate amount of the absorbed moisture.
ii) Appearance verification: Appearance of the powder was verified with the naked eye and using a magnifying lens.
iii) Powder X-ray diffraction measurement: Powder X-ray diffraction patterns of samples before storage and after completion of the storage period were recorded by a powder X-ray diffraction device.
iv) HPLC purity: Detection of degraded products in the samples before storage and after completion of the storage period was carried out using HPLC to measure purity.

(HPLC conditions) Mobile phase: 0.1% trifluoroacetic acid aqueous solution/acetonitrile = 9:2, column: TSKgel ODS 80TsQA, 4.6 x 150 mm, particle diameter 5 µm (mfd. by Tosoh), flow rate: 1 ml/min, column temperature: 40°C, detection: UV 260 nm

### (Results)

### i) Moisture absorption (dehydrated amount): Changes in weight are shown in Table 1.

About 1 % of weight loss was observed in the α-form Crystals under 93% R.H. storage.

Significant weight change was not found in the β-form Crystals under all of the moisture adjustment conditions. ii) Appearance verification:

A change from reddish yellow to yellow was found in the α-form Crystals under 93% R.H. storage. No changes were found under other conditions.

No changes were found in the β-form Crystals under all conditions.

### iii) Powder X-ray diffraction measurement:

Powder X-ray diffraction pattern of the α-form Crystals under 93% R.H. storage showed similar pattern of the β-form Crystals, showing that they were transitioned to β-form Crystals. No changes were found under other conditions.

Powder X-ray diffraction pattern of the β-form Crystals did not change by the storage.

### iv) HPLC purity: The HPLC purity before storage and after 7 days of storage is shown in Table 1.

In each of the α-form Crystals and β-form Crystals, the purity was not decreased and degradation due to storage was not found.

**Table 1**

| Conditions | Bromide α-form Crystals | | Bromide β-form Crystals | |
|---|---|---|---|---|
| | Weight change (%) | HPLC purity (%) | Weight change (%) | HPLC purity (%) |
| Before storage | - | 99.93 | - | 99.72 |
| ca. 0% R.H. | -0.10% | 99.72 | 0.01% | 99.73 |
| 33% R.H. | -0.02% | 99.89 | 0.02% | 99.73 |
| 51% R.H. | -0.04% | 99.89 | 0.04% | 99.72 |
| 75% R.H. | 0.13% | 99.88 | 0.06% | 99.71 |
| 93% R.H. | -0.94% | 99.89 | 0.08% | 99.71 |

Based on the above test results, the α-form Crystals showed no hygroscopic property, and their degradation due to storage was not found, too. Though they transitioned to β-form Crystals in an extremely high moisture environment of 93% R.H. condition, their purity was not reduced, so that it was confirmed that this crystal transition does not accompany degradation.

The β-form Crystals also showed no hygroscopic property, and their degradation due to storage was not found, too. In addition, the crystal transition was not found, too. Thus, it was confirmed that the β-form Crystals are stable physically and chemically under all of the humidity conditions.

Water absorption/desorption behavior of the bromide (β-form Crystal) and the chloride obtained in Reference Example as a comparative compound was measured using a moisture equilibrium measuring device SGA-X100 (VTI) under conditions of temperature: 25°C, measuring range: relative humidity of from 5 to 95%, and measuring interval: 5%.

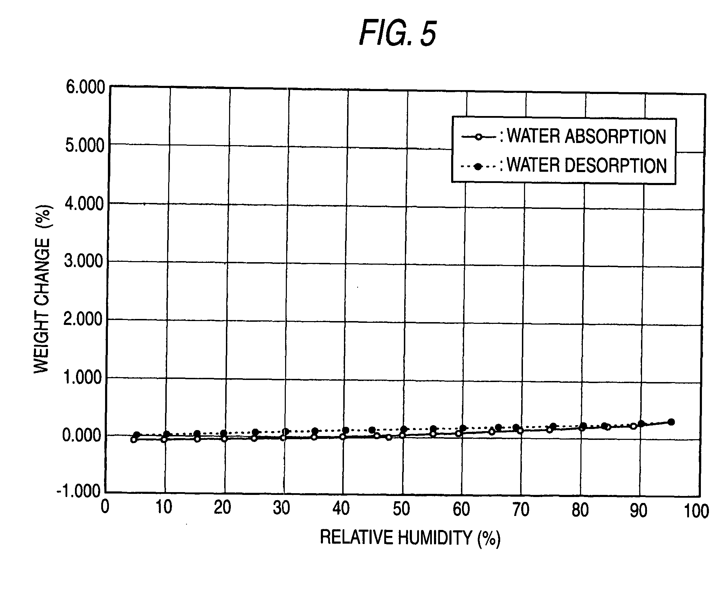

Hygroscopic property was not found in the bromide (β-form Crystal) within the humidity range of from 5 to 95% (cf. Fig. 5).

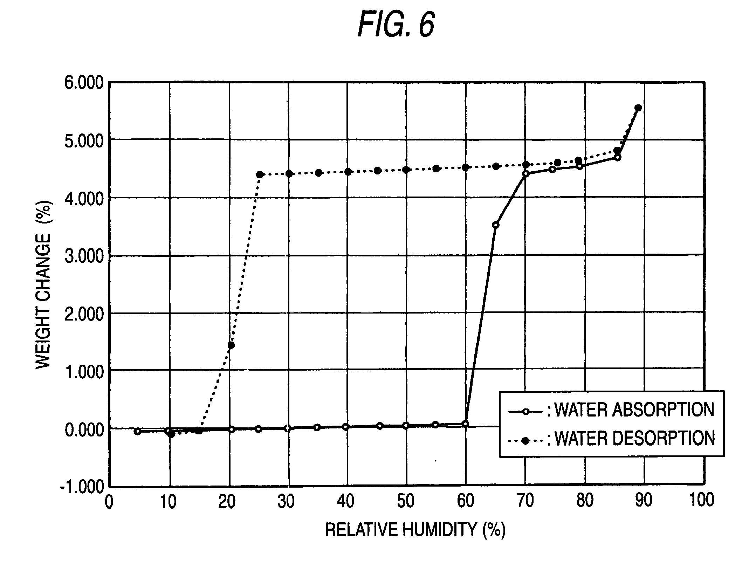

On the other hand, the chloride as the comparative compound absorbed moisture at from 65% R.H. to 80% R.H. and formed monohydrate (a weight gain of 4.3%), and then caused deliquescence at 85% R.H. or more. At the time of drying, this hydration state was stably present up to 25% R.H. and returned to anhydride at lower than that (cf. Fig. 6). In addition, this was also returned to anhydride by a slight heating (about 25°C or more). It was confirmed that the crystallinity is reduced by passing through this moisture absorption and drying and changed such that the moisture absorption starts at a more lower humidity. It was considered that strict moisture control is necessary for the storage of said chloride due to such properties which can be generated within the daily humidity range.

### Test Example 2 (Stability test)

Samples were stored under shade for 3 months in a constant temperature constant moisture vessel of 40°C and 75% R.H. The purity of each sample before storage and after storage was measured using HPLC, and change of crystal form was measured by powder X-ray diffraction. In addition, as a comparative example, crystals of the chloride were stored for 3 months under the same conditions, and amount of an impurity a (a compound in which the imidazolium ring was ring-opened) was measured.

### (Results)

The results are shown in the following table.

**Table 2**

| | | | HPLC purity (%) | Powder X-ray diffraction pattern | Impurity a (%) |
|---|---|---|---|---|---|
| Bromide *1 | α-form Crystal | Before storage | 99.91 | α-form | ND |
| | | After storage | 99.92 | β-form | ND |
| | β-form Crystal | Before storage | 99.71 | β-form | ND |
| | | After storage | 99.71 | β-form | ND |
| Comparative example Chloride crystal *2 | | Before storage | - | - | 0.64 |
| | | After storage | - | - | 2.45 |

| | | | | | |
|---|---|---|---|---|---|
| ND: Not detected 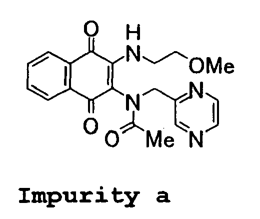 *1 HPLC conditions mobile phase: (solution A) 0.1% trifluoroacetic acid aqueous solution/acetonitrile = 9:2, (solution B) acetonitrile, gradient % B: 0% (0 - 50 min)/linear 60% (50 - 70 min)/60% (70 - 80 min)/0% (80 - 100 min), column: TSKgel ODS 80TsQA, 4.6 x 150 mm, particle diameter 5 µm (mfd. by Tosoh), flow rate: 1 ml/min, column temperature: 40°C, detection: UV 260 nm *2 HPLC conditions mobile phase: (solution A) 0.01 M ammonium acetate aqueous solution (pH 5.0) + 1% acetonitrile, (solution B) acetonitrile, gradient % B: 17% (0 - 4 min)/linear gradient 20% (4 - 10 min), column: Develosil ODS-HG 2.0 x 50 mm, particle diameter 3 µm (mfd. by Nomura Kagaku), flow rate: 0.6 ml/min, column temperature: 40°C, detection: MS (SIM: M/z = 381, polarity: positive) | | | | | |

Transition to the β-form Crystal was observed in the α-form Crystal of the bromide, but degradation due to the storage was not observed. The β-form Crystal of the bromide showed no changes before and after the storage and was very stable physically and chemically.

Contrary to this, storage stability of the already known chloride was low, because the amount of an impurity resulting from ring-opening of the imidazolium ring was evidently increased by the storage.

### Industrial Applicability

The 1-(2-methoxyethyl)-2-methyl-4,9-dioxo-3-(pyrazin-2-ylmethyl)-4,9-dihydro-1H-naphtho[2,3-d]imidazol-3-ium bromide of the invention has no hygroscopic property and is excellent in the stability at the time of storage, so that this is useful as a bulk for medicines. Particularly, its polymorphic α- and β-form Crystals are superior in the storage stability. Among them, the β-form Crystal is most stable crystal and markedly useful as a bulk for medicines.

## Claims

1. A crystal of 1-(2-methoxyethyl)-2-methyl-4, 9-dioxo-3-(pyrazin-2-ylmethyl)-4,9-d ihydro-1H-naphtho [2,3-d] imidazol-3-ium bromide, wherein it shows _(a) the peaks of 2θ (°) = 8.5, 14.8, 19.7, 25.7 and 30.2 by powder X-ray diffraction, or (b) the peaks of 2θ (°) = 9.2, 12.6, 14.6, 18.0, 21.1, 24.9, 26.4 and 27.1 by powder X-ray diffraction, wherein X-ray diffraction measurement uses a Cu X-ray tube and a wavelength of 1.54056 A.

2. The crystal described in claim 1, wherein it shows the peaks of 2θ (°) = 8.5, 14.8, 19.7, 25.7 and 30.2 by powder X-ray diffraction.

3. The crystal described in claim 1, wherein it shows the peaks of 2θ (°) = 9.2, 12.6, 14.6, 18.0, 21.1, 24.9, 26.4 and 27.1 by powder X-ray diffraction.

4. The crystal described in claim 1, wherein it shows a heat absorption peak at from 207 to 213°C by DSC analysis.

5. The crystal described in claim 1, wherein it shows a heat absorption peak at from 201 to 207°C by DSC analysis.

## Patentansprüche

1. Kristall aus 1-(2-Methoxyethyl)-2-methyl-4,9-dioxo-3-(pyrazin-2-ylmethyl)-4,9-dihydro-1H-naphtho[2,3-d]imidazol-3-iumbromid, der Folgendes aufweist: (a) die Spitzen von 2θ (°) = 8,5, 14,8, 19,7, 25,7 und 30,2 durch Pulverröntgenbeugung, oder (b) die Spitzen von 2θ (°) = 9,2, 12,6, 14,6, 18,0, 21,1, 24,9, 26,4 und 27,1 durch Pulverröntgenbeugung, wobei zur Röntgenbeugungsmessung eine Cu-Röntgenstrahlenröhre und eine Wellenlänge von 1,54056 A benutzt werden.

2. Kristall nach Anspruch 1, der die Spitzen von 2θ (°) = 8,5, 14,8, 19,7, 25,7 und 30,2 durch Pulverröntgenbeugung aufweist.

3. Kristall nach Anspruch 1, der die Spitzen von 2θ (°) = 9,2, 12, 6, 14, 6, 18,0, 21,1, 24, 9, 26,4 und 27,1 durch Pulverröntgenbeugung aufweist.

4. Kristall nach Anspruch 1, der eine Wärmeabsorptionsspitze bei 207 bis 213°C durch DSC-Analyse aufweist.

5. Kristall nach Anspruch 1, der eine Wärmeabsorptionsspitze bei 201 bis 207°C durch DSC-Analyse aufweist.

## Revendications

1. Cristal de bromure de 1-(2-méthoxyéthyl)-2-méthyl-4, 9-dioxo-3-(pyrazin-2-yméthyl)-4,9-dihydro-1 H-naphtho [2,3-d] imidazol-3-ium, montrant (a) les pics de 2θ(°) = 8,5, 14,8, 19,7, 25,7 et 30,2 par diffraction des rayons X sur poudre, ou (b) les pics de 2θ(°) = 9,2, 12,6, 14,6, 18,0, 21,1, 24,9, 26,4 et 27,4 par diffraction des rayons X sur poudre, la mesure de diffraction des rayons X utilisant un tube de rayons X en Cu et une longueur d'onde de 1,54056A.

2. Cristal selon la revendication 1, montrant les pics de 2θ(°) = 8,5, 14,8, 19,7, 25,7 et 30,2 par diffraction des rayons X sur poudre.

3. Cristal selon la revendication 1, montrant les pics de 2θ(°) = 9,2, 12,6, 14,6 , 18,0, 21,1, 24,9, 26,4 et 27,1 par diffraction des rayons X sur poudre.

4. Cristal selon la revendication 1, montrant un pic d'absorption de chaleur de 207 à 213°C par analyse DCS.

5. Cristal selon la revendication 1, montrant un pic d'absorption de chaleur de 201 à 207°C par analyse DCS.
